# EUROPÄISCHE PATENTSCHRIFT

(11) **EP 0 317 888 B1**
(45) Veröffentlichungstag und Bekanntmachung des Hinweises auf die Patenterteilung: **24.03.1993**
(21) Anmeldenummer: 88119077.1
(22) Anmeldetag: 17.11.1988
(51) Int. Cl.: C07C 205/44, C07C 205/51, C07C 309/65, C07C 255/03, C07C 205/08, C07C 205/45

(54) **Neue fluorierte Nitroalkylverbindungen und ein Verfahren zur Herstellung von neuen und bekannten fluorierten Nitroalkylverbindungen**
Fluorinated nitroalkyl compounds, and process for the preparation of fluorinated nitroalkyl compounds
Composés nitroalkyl fluorés ainsi qu'un procédé pour la préparation de composés nitroalkyl fluorés

(30) Priorität: 24.11.1987 DE 3739784
(43) Veröffentlichungstag der Anmeldung: 31.05.1989
(73) Patentinhaber: BAYER AG, 51368 Leverkusen (DE)
(72) Erfinder: Negele, Michael, Dr., D-5000 Köln 80 (DE); Baasner, Bernd, Dr., D-5090 Leverkusen (DE); Marhold, Albrecht, Dr., D-5090 Leverkusen (DE)

(56) Entgegenhaltungen:
- DE-A- 3 808 276
- TETRAHEDRON, vol. 26, 1970, pages 5737-5743, Pergamon Press, GB; E.R. BISSELL et al.: "Fluorine-containing nitrogen compounds-VI. Sulfur tetrafluoride fluorination of aliphatic nitro acids"
- CHEMISTRY LETTERS, 1983, pages 1145-1148, The Chemical Society of Japan; J.H. CLARK et al.: "Fluoride ion catalysed Michael reactions"
- JOURNAL ORGANIC CHEMISTRY, vol. 51, no. 12, June 1986, pages 2366-2368, American Chemical Society, Washington, US; H. OGOSHI et al.: "1,1,1-trifluoro-2-penten-4-one as a building block of trifluoromethyl-substituted compounds"

## Beschreibung

Die vorliegende Erfindung betrifft neue fluorierte Nitroalkylverbindungen und ein Verfahren zur Herstellung der neuen und bekannten fluorierten Nitroalkylverbindungen aus Fluornitroalkanen und α,β-ungesättigten Verbindungen.

Es sind bisher zur zwei fluorierte Nitroalkylverbindungen des Typs 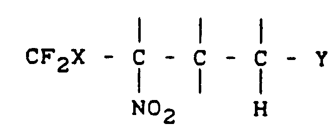
bekanntgeworden, nämlich 4-Nitro-4,5,5,5-tetrafluorpentan-carbonsäuremethylester und 4-Nitro-4-trifluormethyl-pentan-carbonsäuremethylester. 4-Nitro-4-trifluormethyl-pentan-carbonsäuremethylester ist aus Acrylsäuremethylester und CF₃-CNO₂(CH₃)-H in Substanz in Gegenwart von Kaliumfluorid als Base hergestellt worden, 4-Nitro-4,5,5,5-tetrafluorpentan-carbonsäuremethylester auf andere Weise (siehe Tetrahedron 26, 5737 (1970). Nachteilig bei diesem Verfahren sind die benötigten langen Reaktionszeiten und die schlechten Ausbeuten.

Es wurden nun fluorierte Nitroalkylverbindungen der Formel (I) gefunden 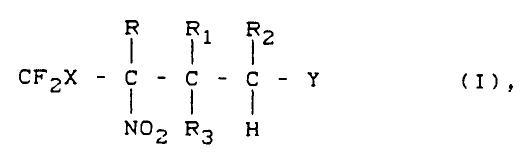
in der
- X: für Fluor oder Wasserstoff steht,
- R: für Wasserstoff, Halogen oder C₁- bis C₆-Alkyl steht
- R₁, R₂ und R₃: unabhängig voneinander jeweils für Wasserstoff, Halogen, Cyano, gegebenenfalls substituiertes C₁- bis C₆-Alkyl oder gegebenenfalls substituiertes C₆- bis C₁₀-Aryl stehen, wobei R₁ und R₂ gemeinsam mit den C-Atomen, an die sie gebunden sind, auch einen gegebenenfalls substituierten gesättigten C₅- bis C₇-carbocyclischen Ring bilden können und
- Y: für eine -CO-R₄-Gruppe steht mit R₄ = Wasserstoff, C₁- bis C₆-Alkyl oder C₆- bis C₁₀-Aryl, wobei R₄ und R₃ gemeinsam mit den dazwischen befindlichen C-Atomen auch einen gegebenenfalls substituierten gesättigten C₅- bis C₇-carbocyclischen Ring bilden können oder
für eine -CO-OR₅-Gruppe steht mit R₅ = C₁- bis C₆-Alkyl oder C₆- bis C₁₀-Aryl oder
für eine -SO₂-OR₆-Gruppe steht mit R₆ = C₁- bis C₆-Alkyl oder C₆- bis C₁₀-Aryl oder
für eine Cyanogruppe steht oder
für eine Nitrogruppe steht,
wobei die Substituentenkombinationen
X = F, R = F, R₁ bis R₃ = H, Y = -CO-OCH₃ und
X = F, R = CH₃, R₁ bis R₃ = H, Y = -CO-OCH₃
ausgenommen sind.

Bei den erfindungsgemäßen fluorierten Nitroalkylverbindungen der Formel (I) kann es sich jeweils um eine Verbindung in einer optisch aktiven Form handeln, aber auch um beliebige Gemische verschiedener optisch aktiver Formen einer Verbindung. Beispielsweise ist folgendes möglich: Wenn in Formel (I) R₁, R₂ und R₃ für Wasserstoff stehen oder R₁ und R₂ Teil eines unsubstituierten oder symmetrisch substituierten Ringes sind, kann die jeweilige Verbindung in der R-Form, der S-Form und in beliebigen Mischungen dieser Formen vorliegen. Wenn in Formel (I) R₁ und R₃ verschieden sind und R₂ für Wasserstoff steht oder R₁ und R₂ Teil eines unsymmetrisch substituierten Ringes sind, kann die jeweilige Verbindung in der R,R-, R,S-, S,R- oder S,S-Form vorliegen oder in beliebigen Mischungen von zwei, drei oder allen diesen Formen. Wenn in Formel (I) R₁ und R₃ verschieden sind und R₂ nicht für Wasserstoff steht, kann die jeweilige Verbindung in der R,R,R-, R,R,S-, R,S,R-, S,R,R-, S,S,R-, S,R,S-, R,S,S- oder S,S,S-Form oder in beliebigen Mischungen von zwei oder mehr oder allen diesen Formen vorliegen.

Soweit in Formel (I) R₁, R₂ und/oder R₃ für einen substituierten C₁- bis C₆-Alkylrest oder einen substituierten C₆- bis C₁₀-Arylrest stehen oder R₁ und R₂ oder R₃ und R₄ Teil eines substituierten gesättigten C₅- bis C₇-carbocyclischen Ringes sind, kommen als Substituenten an den Alkylresten, Arylresten und/oder gesättigten Ringen beispielsweise Nitro-, Halogen-, C₁-bis C₄-Alkoxy und/oder CN-Gruppen in Frage. Vorzugsweise steht Halogen dabei für Fluor, Chlor oder Brom, insbesondere für Fluor oder Chlor. C₁- bis C₄-Alkoxygruppen stehen dabei vorzugsweise für Methoxy-, Ethoxy- oder Propoxy-Gruppen. Als Substituenten an Alkylresten und/oder gesättigten Ringen kommen auch gegebenenfalls substituierte aromatische Reste in Frage.

In Formel (I) stehen unabhängig voneinander X vorzugsweise für Fluor, R vorzugsweise für Wasserstoff oder Methyl, R₁ vorzugsweise für Wasserstoff, Methyl oder Phenyl, R₂ vorzugsweise für Wasserstoff, Methyl, Fluor, Chlor, Brom, CN oder Phenyl, R₃ vorzugsweise für Wasserstoff, Methyl oder Phenyl. Steht in Formel (I) Y für eine -CO-R₄-Gruppe, so ist Y vorzugsweise eine -CO-H-, -COCH₃-, -COC₂H₅- oder -CO-Phenyl-Gruppe. Steht in Formel (I) Y für eine -CO-OR₅-Gruppe, so ist Y vorzugsweise eine -CO-OCH₃-, -CO-OC₂H₅- oder -CO-O-n-Butyl-Gruppe. Steht in Formel (I) Y für -SO₂-OR₆-, so ist Y vorzugsweise eine -SO₂-OCH₃-, -SO₂-OC₂H₅- oder -SO₂-O-. Phenyl-Gruppe. Außerdem ist bevorzugt, daß Y für eine -CO-R₄-Gruppe steht und R₃ gemeinsam mit dem Carbonylkohlenstoff der -CO-R₄-Gruppe und den dazwischenliegenden C-Atomen einen unsubstituierten, gesättigten C₅-oder C₆-Ring bilden.

Besonders bevorzugt sind Verbindungen der Formel (I), bei denen unabhängig voneinander X für Fluor, R für Wasserstoff oder Methyl, R₁ für Wasserstoff oder Methyl, R₂ und R₃ für Wasserstoff und Y für -COCH₃, -CN, -CO-OCH₃, -COH, -SO₂OCH₃ oder -NO₂ stehen.

Besonders bevorzugte Verbindungen der Formel (I) haben folgende Substituenten (siehe Tabelle 1).

**Tabelle 1**

| X | R | R₁ | R₂ | R₃ | Y |
|---|---|---|---|---|---|
| F | H | H | H | H | CO-CH₃ |
| F | H | H | H | H | CO-OCH₃ |
| F | H | H | H | H | CN |
| F | CH₃ | H | H | H | CO-CH₃ |
| F | CH₃ | H | H | H | CO-H |
| F | CH₃ | H | H | H | CO-OCH₃ |
| F | CH₃ | H | H | H | CN |
| F | CH₃ | CH₃ | H | H | CO-H |
| F | CH₃ | H | H | H | SO₂-OCH₃ |
| F | CH₃ | H | H | H | NO₂ |

Weiterhin wurde ein Verfahren zur Herstellung von fluorierten Nitroalkylverbindungen der Formel (Ia) gefunden 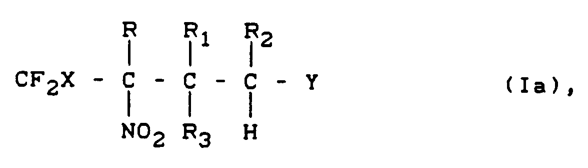
in der
- X: für Fluor oder Wasserstoff steht,
- R: für Wasserstoff, Halogen oder C₁- bis C₆-Alkyl steht,
- R₁, R₂ und R₃: unabhängig voneinander jeweils für Wasserstoff, Halogen, Cyano, gegebenenfalls substituiertes C₁- bis C₆-Alkyl oder gegebenenfalls substituiertes C₆- bis C₁₀-Aryl stehen, wobei R₁ und R₂ gemeinsam mit den C-Atomen, an die sie gebunden sind, auch einen gegebenenfalls substituierten gesättigten C₅- bis C₇-carbocyclischen Ring bilden können und
- Y: für eine -CO-R₄-Gruppe steht mit R₄ = Wasserstoff, C₁- bis C₆-Alkyl oder C₆- bis C₁₀-Aryl, wobei R₄ und R₃ gemeinsam mit den dazwischen befindlichen C-Atomen auch einen gegebenenfalls substituierten gesättigten C₅- bis C₇-carbocyclischen Ring bilden können oder
für eine -CO-OR₅-Gruppe steht mit R₅ = C₁- bis C₆-Alkyl oder C₆- bis C₁₀-Aryl oder
für eine -SO₂-OR₆-Gruppe steht mit R₆ = C₁- bis C₆-Alkyl oder C₆- bis C₁₀-Aryl oder
für eine Cyanogruppe steht oder
für eine Nitrogruppe steht,
das dadurch gekennzeichnet ist, daß man ein Fluornitroalkan der Formel (II) 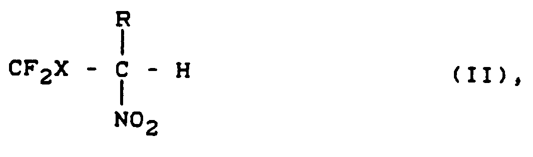
in der
X und R die bei Formel (Ia) angegebene Bedeutung haben, mit α,β-ungesättigten Verbindungen der Formel (III) 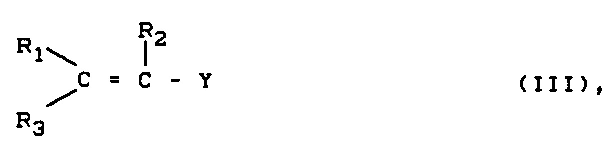
in der
R₁, R₂, R₃ und Y die bei Formel (Ia) angegebene Bedeutung haben,
in Gegenwart von Alkalimetallfluoriden und einem polaren, aprotischen Lösungsmittel umsetzt.

Es wird ausdrücklich darauf hingewiesen, daß sich das erfindungsgemäße Herstellungsverfahren auch auf die Herstellung der Verbindungen der Formel (Ia) erstreckt, bei denen die Substituentenkombinationen X = F, R = F, R₁ bis R₃ = H, Y = -CO-OCH₃ und X = F, R = CH₃, R₁ bis R₃ = H, Y = -CO-OCH₃ vorliegen.

Fluornitroalkane der Formel (II) sind bekannt und können beispielsweise hergestellt werden wie es in den DE-OS'en 33 05 201 und 33 05 202 beschrieben ist, α,β-Ungesättigte Verbindungen der Formel (III) sind ebenfalls bekannt, gut zugänglich und zum Teil im Handel erhältlich.

Bevorzugte und besonders bevorzugte Verbindungen der Formeln (II) und (III) sind solche, bei denen die Substituenten die bei der Erörterung der Formel (I) als bevorzugt und besonders bevorzugt angegebenen Bedeutungen haben. Bei den Verbindungen der Formel (III) sind beispielsweise Acrylsäuremethylester, Methylvinylketon, Acrylnitril, Acrolein, Crotonaldehyd, Vinylsulfonsäuremethylester und Nitroethylen bevorzugt.

Falls in Formel (II) R für Wasserstoff steht, können in unerwünschter Weise auch 2 Mole der Verbindung der Formel (III) mit 1 Mol der Verbindung der Formel (II) reagieren. In diesen Fällen ist es deshalb bevorzugt, die Verbindung der Formel (II) im Überschuß einzusetzen, beispielsweise 3 bis 5 Mole der Verbindung der Formel (II) auf 1 Mol der Verbindung der Formel (III).

Falls in Formel (II) R verschieden von Wasserstoff ist, ist es bevorzugt, die Verbindung der Formel (III) im Überschuß einzusetzen, beispielsweise 1,2 bis 3 Mole der Verbindung der Formel (III) auf 1 Mol der Verbindung der Formel (II).

Das erfindungsgemäße Verfahren kann beispielsweise bei Temperaturen durchgeführt werden, die zwischen -20°C und dem Siedepunkt (bei Normaldruck) der am niedrigsten siedenden Komponente des Reaktionsgemisches liegen. Bevorzugt liegt die Reaktionstemperatur im Bereich 0 bis 25°C.

Im allgemeinen wird das erfindungsgemäße Verfahren bei Normaldruck durchgeführt. Es kann jedoch auch bei erniedrigtem oder erhöhtem Druck durchgeführt werden, beispielsweise bei Drucken im Bereich von 0,5 bar bis 15 kbar.

Als polare, aprotische Lösungsmittel sind beispielsweise aliphatische Carbonsäurenitrile, N,N-Dialkyl-substituierte Carbonsäureamide, Dialkylsulfoxide, Tetrahydrothiophen-1,1-dioxid, Tetraalkylharnstoffe und/oder Dialkylether des Ethylenglykols und des Diethylenglykols geeignet. Besonders bevorzugt wird Acetonitril als Lösungsmittel verwendet.

Als Alkalimetallfluoride kommen beispielsweise Natriumfluorid, Kaliumfluorid, Rubidiumfluorid und/oder Cäsiumfluorid in Frage. Vorzugsweise wird Kaliumfluorid und/oder Cäsiumfluorid, besonders bevorzugt Kaliumfluorid eingesetzt. Die Alkalimetallfluoride können dabei in technischer oder chemisch reiner Form und wasserfrei oder wasserhaltig zum Einsatz gelangen. Vorzugsweise wird Kaliumfluorid in chemisch reiner, wasserfreier Form verwendet. Die Alkalimetallfluoride können beispielsweise in Mengen von 0,1 bis 100 Mol-% bezogen auf die Verbindung der Formel (II) eingesetzt werden. Vorzugsweise beträgt diese Menge 1 bis 50 Mol-%.

In einer besonderen Ausführungsform des erfindungsgemäßen Verfahrens wird zusätzlich in Gegenwart von Kronenethern gearbeitet. Durch Zusätze von Kronenethern kann häufig die Löslichkeit der Alkalimetallfluoride in polaren aprotischen Lösungsmitteln verbessert werden. Als Kronenether kommt beispielsweise 1,4,7,10,13,16-Hexaoxacyclooctadecan (18-Krone-6) in Frage. Kronenether können beispielsweise in Mengen von 1 bis 3 Mol-% (bezogen auf eingesetzte Alkalimetallfluoride) verwendet werden.

In einer weiteren speziellen Ausführungsform des erfindungsgemäßen Verfahrens wird in Gegenwart von Tetraalkylammoniumfluoriden gearbeitet. Beispielsweise kommt hierfür Tetrabutylammoniumfluorid, Tetraethylammoniumfluorid und/oder Butyltrimethylammoniumfluorid in Frage. Tetraalkylammoniumfluoride können beispielsweise in Mengen von 0,5 bis 10 Mol-% [bezogen auf die Verbindung der Formel (II)] eingesetzt werden.

Gemäß einer weiteren speziellen Ausführungsform des erfindungsgemäßen Verfahrens können die Alkalimetallfluoride aufgebracht auf anorganische oxidische Träger eingesetzt werden. Als oxidische Träger kommen beispielsweise Siliciumdioxid und/oder Aluminiumoxid in Frage (siehe z.B. J. H. Clark, Chem. Rev. 80, 429 (1980) und Tetrahedron Lett. 28, 1421 (1987)). Bei dieser Variante des erfindungsgemäßen Verfahrens kommt vorzugsweise Kaliumfluorid aufgebracht auf basischem Aluminiumoxid zum Einsatz, dessen Herstellung beispielsweise in J. Org. Chem. 52, 1601 (1987) beschrieben ist.

Es ist vorteilhaft, während der erfindungsgemäßen Umsetzung zu rühren. Die Reaktionszeit kann beispielsweise zwischen 30 Minuten und 16 Stunden liegen, bevorzugt ist eine Reaktionszeit im Bereich von 2 bis 6 Stunden.

Das nach der erfindungsgemäßen Umsetzung vorliegende Reaktionsgemisch kann beispielsweise wie folgt aufgearbeitet werden:
Man trennt zunächst die festen Bestandteile des Reaktionsgemisches durch Filtration ab und wäscht die isolierten festen Bestandteile mit einem mit Wasser nicht mischbaren Lösungsmittel, z.B. Dichlormethan, nach. Danach setzt man stark verdünnte wäßrige Salzsäure zu, um gegebenenfalls gelöste basische Salze zu entfernen. Nach Abtrennung der wäßrigen Phase entfernt man aus der organischen Phase das Lösungsmittel und gegebenenfalls vorhandene nicht umgesetzte Ausgangsprodukte der Formel (II) und/oder der Formel (III), z.B. durch Abdampfen und/oder Destillation, gegebenenfalls bei vermindertem Druck. Es verbleiben dann die Produkte der Formel (Ia) in einer Reinheit, die für viele Verwendungen völlig ausreichend ist. Gegebenenfalls kann man die so erhaltenen Verbindungen der Formel (Ia) durch eine fraktionierte Destillation bei vermindertem Druck noch weiter reinigen.

Die erfindungsgemäß zugänglichen neuen und bekannten fluorierten Nitroalkylverbindungen der Formel (Ia) können als Zwischenprodukte zur Herstellung von biologisch aktiven fluorierten Aminoalkylverbindungen und biologisch aktiven fluorierten 5-gliedrigen Stickstoffheterocyclen verwendet werden. Derartige biologisch aktive Verbindungen können auf an sich bekannte Weise (siehe z.B. S. L. Ioffe et al., Russ. Chem. Rev. 35, 19 (1966)) aus den Verbindungen der Formel (Ia) erhalten werden. Die biologisch aktiven fluorierten Aminoalkylverbindungen und fluorierten 5-gliedrigen Stickstoffheterocyclen können als Pharmazeutika und/oder Pflanzenschutzmittel verwendet werden.

Das erfindungsgemäße Verfahren zur Herstellung fluorierter Nitroalkylverbindungen der Formel (Ia) hat gegenüber dem bisher bekannten Verfahren zur Herstellung von 4-Nitro-4-trifluormethyl-pentan-carbonsäuremethylester deutliche Vorteile. So sind die Reaktionszeiten stark verkürzt und die Ausbeuten deutlich besser. Keinesfalls war voraussehbar, daß sich das erfindungsgemäße Verfahren mit einer Vielzahl α,β-ungesättigter Verbindungen der Formel (III) durchführen läßt und so neue fluorierte Nitroalkylverbindungen der Formel (I) auf einfache Weise zugänglich werden. Darüber hinaus ist überraschend, daß nach dem erfindungsgemäßen Verfahren die fluorierten Nitroalkylverbindungen der Formel (Ia) auf einfache Weise isoliert werden können und sonst nötige aufwendige Reinigungsschritte, beispielsweise präparative Gaschromatographie oder präparative Hochdruckflüssigkeitschromatographie nicht notwendig sind.

### Beispiele

### Allgemeine Arbeitsweise A:

In einem 250 ml Rundkolben wurden in 80 ml Acetonitril 200 mMol des jeweils angegebenen Fluornitroalkans der Formel (II) mit X = Fluor und R = H und 50 mMol Kaliumfluorid bei 0 bis 5°C vorgelegt und innerhalb von 30 Minuten unter Rühren mit 180 mMol der jeweils angegebenen α,β-ungesättigten Verbindung der Formel (III) mit R₂ und R₃ = Wasserstoff versetzt. Nach dem Abklingen der schwach exothermen Reaktion wurde das Kühlbad entfernt und bei 20°C 3 Stunden nachgerührt. Zur Aufarbeitung wurde das Reaktionsgemisch filtriert, das Kaliumfluorid mit 150 ml Dichlormethan nachgewaschen und die vereinigten Filtrate mit 100 ml 2 gew.-%iger wäßriger Salzsäure gewaschen. Nach dem Trocknen über Natriumsulfat wurde am Rotationsverdampfer im Wasserstrahlvakuum bei 40°C Badtemperatur eingeengt. Der ölige Rückstand wurde im Vakuum einer Ölpumpe destilliert und so die jeweils angegebene Verbindung der Formel (I) mit X= Fluor und R₂ und R₃ = Wasserstoff erhalten.

### Allgemeine Arbeitsweise B:

In einem 250 ml Rundkolben wurden in 80 ml Acetonitril 300 mMol der jeweils angegebenen α,β-ungesättigten Verbindung der Formel (III) und 50 mMol Kaliumfluorid bei 0 bis 5°C vorgelegt und innerhalb von 30 Minuten unter Rühren 200 mMol des jeweils angegebenen Fluornitroalkans der Formel (II) mit R ≠ Wasserstoff versetzt. Nach Abklingen der schwach exothermen Reaktion wurde das Kühlbad entfernt und bei 20°C 3 Stunden nachgerührt. Die weitere Aufarbeitung erfolgte wie bei der allgemeinen Arbeitsweise A, wobei das Produkt bei Beispiel 10 jedoch nicht destilliert wurde.

### Beispiele 1 bis 10

Die Einsatzprodukte, Reaktionsprodukte, deren Charakterisierung und die jeweils angewendete Arbeitsweise ist aus der folgenden Tabelle 2 ersichtlich.

Die Ausbeuten der Beispiele 1 bis 9 beziehen sich auf ein gereinigtes Produkt, das nach einmaliger Destillation erhalten wurde. Im Beispiel 10 ist die Rohausbeute nach Entfernen des Lösungsmittels angegeben. Alle Ausbeuten beziehen sich auf die im Unterschuß eingesetzte Reaktionskomponente.

Die ¹H-NMR- und ¹⁹F-NMR-Spektren bestätigen die angegebenen Strukturen.

**Tabelle 2**

| Beispiel Nr. | Bedeutung von R im Ausgangsprodukt der Formel (II) mit X = Fluor | Bedeutung von R₁ und Y im Ausgangsprodukt der Formel (III) mit R₂ und R₃ = Wasserstoff | | Allgemeine Arbeitsweise | Ausbeute und Siedepunkte der erhaltenen Produkte der Formel (I) mit Substituenten entsprechend den eingesetzten Verbindungen der Formel (II) und (III) | |
|---|---|---|---|---|---|---|
| | | R₁ | Y | | | |
| 1 | H | H | -CO-OCH₃ | A | 17 % | 43-45°C/0,6 mbar |
| 2 | H | H | -CO-CH₃ | A | 35 % | 45-50°C/0,6 mbar |
| 3 | H | H | -CN | A | 43 % | 70-72°C/0,2 mbar |
| 4 | CH₃ | H | -CO-OCH₃ | B | 61 % | 55-61°C/0,6 mbar |
| 5 | CH₃ | H | -CO-CH₃ | B | 73 % | 55-57°C/0,6 mbar |
| 6 | CH₃ | H | -CN | B | 84 % | 77-78°C/0,15 mbar |
| 7 | CH₃ | H | -CO-H | B | 30 % | 49-53°C/0,9 mbar |
| 8 | CH₃ | CH₃ | -CO-H | B | 47 %^{*)} | 62-64°C/0,6 mbar |
| 9 | CH₃ | H | -SO₂-OCH₃ | B | 22 % | 82-86°C/0,2 mbar |
| 10 | CH₃ | H | -NO₂ | B | 26 % | Öl, nicht destillierbar |

| | | | | | | |
|---|---|---|---|---|---|---|
| ^{*)} Diastereoisomerenverhältnis gemäß ¹H-NMR-Spektrum 70 Gew.-% : 30 Gew.-% | | | | | | |

## Patentansprüche

1. Fluorierte Nitroalkylverbindungen der Formel (I) 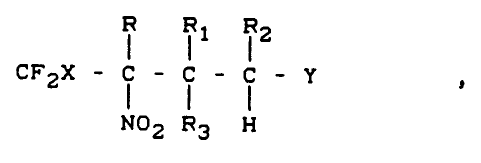 in der
X für Fluor oder Wasserstoff steht,
R für Wasserstoff, Halogen oder C₁- bis C₆-Alkyl steht
R₁, R₂ und R₃ unabhängig voneinander jeweils für Wasserstoff, Halogen, Cyano, gegebenenfalls substituiertes C₁- bis C₆-Alkyl oder gegebenenfalls substituiertes C₆- bis C₁₀-Aryl stehen, wobei R₁ und R₂ gemeinsam mit den C-Atomen, an die sie gebunden sind, auch einen gegebenenfalls substituierten gesättigten C₅-bis C₇-carbocyclischen Ring bilden können und
Y für eine -CO-R₄-Gruppe steht mit R₄ = Wasserstoff, C₁- bis C₆-Alkyl oder C₆- bis C₁₀-Aryl, wobei R₄ und R₃ gemeinsam mit den dazwischen befindlichen C-Atomen auch einen gegebenenfalls substituierten gesättigten C₅- bis C₇-carbocyclischen Ring bilden können oder
für eine -CO-OR₅-Gruppe steht mit R₅ = C₁- bis C₆-Alkyl oder C₆- bis C₁₀-Aryl oder
für eine -SO₂-OR₆-Gruppe steht mit R₆ = C₁-bis C₆-Alkyl oder C₆- bis C₁₀-Aryl oder
für eine Cyanogruppe steht oder
für eine Nitrogruppe steht,
wobei die Substituentenkombinationen
X = F, R = F, R₁ bis R₃ = H, Y = -CO-OCH₃ und
X = F, R = CH₃, R₁ bis R₃ = H, Y = -CO-OCH₃
ausgenommen sind.

2. Fluorierte Nitroalkylverbindungen gemäß Anspruch 1, dadurch gekennzeichnet, daß in Formel (I) unabhängig voneinander X für Fluor, R für Wasserstoff oder Methyl, R₁ für Wasserstoff, Methyl oder Phenyl, R₂ für Wasserstoff, Methyl, Fluor, Chlor, Brom, CN oder Phenyl, R₃ für Wasserstoff, Methyl oder Phenyl und Y für eine -CO-H-, -CO-CH₃-, -COC₂H₅-, -CO-Phenyl-, -CO-OCH₃-, -CO-OC₂H₅-, -CO-O-n-Butyl-, -SO₂-OCH₃-, -SO₂-OC₂H₅- oder -SO₂-O-Phenyl-Gruppe stehen.

3. Fluorierte Nitroalkylverbindungen gemäß Anspruch 1, dadurch gekennzeichnet, daß in Formel (I) Y für eine -CO-R₄-Gruppe steht und R₃ gemeinsam mit dem Carbonylkohlenstoff der -CO-R₄-Gruppe und den dazwischenliegenden C-Atomen einen unsubstituierten, gesättigten C₅- oder C₆-Ring bilden.

4. Fluorierte Nitroalkylverbindungen gemäß Ansprüchen 1 oder 2, dadurch gekennzeichnet, daß in Formel (I) X für Fluor, R für Wasserstoff oder Methyl, R₁ für Wasserstoff oder Methyl, R₂ und R₃ für Wasserstoff und Y für -CO-CH₃, -CN, -CO-OCH₃, -CO-H, -SO₂-OCH₃ oder -NO₂ stehen.

5. Verfahren zur Herstellung von fluorierten Nitroalkylverbindungen der Formel (Ia) 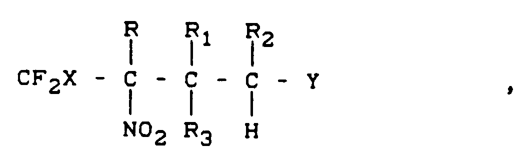 in der
X für Fluor oder Wasserstoff steht,
R für Wasserstoff, Halogen oder C₁- bis C₆-Alkyl steht,
R₁, R₂ und R₃ unabhängig voneinander jeweils für Wasserstoff, Halogen, Cyano, gegebenenfalls substituiertes C₁- bis C₆-Alkyl oder gegebenenfalls substituiertes C₆- bis C₁₀-Aryl stehen, wobei R₁ und R₂ gemeinsam mit den C-Atomen, an die sie gebunden sind, auch einen gegebenenfalls substituierten gesättigten C₅-bis C₇-carbocyclischen Ring bilden können und
Y für eine -CO-R₄-Gruppe steht mit R₄ = Wasserstoff, C₁- bis C₆-Alkyl oder C₆- bis C₁₀-Aryl, wobei R₄ und R₃ gemeinsam mit den dazwischen befindlichen C-Atomen auch einen gegebenenfalls substituierten gesättigten C₅- bis C₇-carbocyclischen Ring bilden können oder
für eine -CO-OR₅-Gruppe steht mit R₅ = C₁- bis C₆-Alkyl oder C₆- bis C₁₀-Aryl oder
für eine -SO₂-OR₆-Gruppe steht mit R₆ = C₁-bis C₆-Alkyl oder C₆- bis C₁₀-Aryl oder
für eine Cyanogruppe steht oder
für eine Nitrogruppe steht,
dadurch gekennzeichnet, daß man ein Fluornitroalkan der Formel (II) 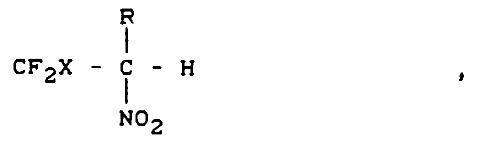 in der
X und R die bei Formel (Ia) angegebene Bedeutung haben, mit α,β-ungesättigten Verbindungen der Formel (III) 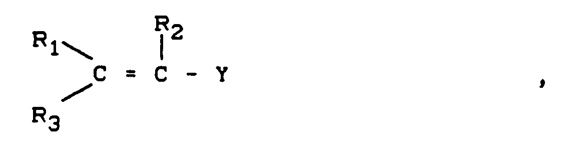 in der
R₁, R₂, R₃ und Y die bei Formel (Ia) angegebene Bedeutung haben,
in Gegenwart von Alkalimetallfluoriden und einem polaren, aprotischen Lösungsmittel umsetzt.

6. Verfahren nach Anspruch 5, dadurch gekennzeichnet, daß man beim Einsatz einer Verbindung der Formel (II) mit R = Wasserstoff 3 bis 5 Mole der Verbindung der Formel (II) auf 1 Mol der Verbindung der Formel (III) einsetzt.

7. Verfahren nach Anspruch 5, dadurch gekennzeichnet, daß man beim Einsatz einer Verbindung der Formel (ii) mit R ≠ Wasserstoff 1,2 bis 3 Mole der Verbindung der Formel (III) auf 1 Mol der Verbindung der Formel (II) einsetzt.

8. Verfahren nach Ansprüchen 5 bis 7, dadurch gekennzeichnet, daß man 0,1 bis 100 Mol-% Alkalimetallfluorid einsetzt [bezogen auf die Verbindung der Formel (II)].

9. Verfahren nach Ansprüchen 5 bis 8, dadurch gekennzeichnet, daß man Kaliumfluorid und/oder Cäsiumfluorid einsetzt.

10. Verfahren nach Ansprüchen 5 bis 9, dadurch gekennzeichnet, daß man bei Temperaturen im Bereich von -20°C bis zum Siedepunkt (bei Normaldruck) der am niedrigsten siedenden Komponente des Reaktionsgemisches arbeitet.

## Claims

1. Fluorinated nitroalkyl compounds of the formula 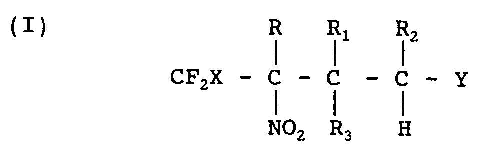 in which
X represents fluoro or hydrogen,
R represents hydrogen, halogen or C₁-C₆-alkyl,
R₁, R₂ and R₃ independently of one another each represent hydrogen, halogen, cyano, substituted or unsubstituted C₁-C₆-alkyl or substituted or unsubstituted C₆-C₁₀-aryl, it also being possible for R₁ and R₂ together with the C atoms to which they are bound to form a substituted or unsubstituted saturated C₅-C₇-carbocyclic ring and
Y represents a -CO-R₄ group where R₄ is hydrogen, C₁-C₆-alkyl or C₆-C₁₀-aryl, it also being possible for R₄ and R₃ together with the C atoms in between to form a substituted or unsubstituted saturated C₅-C₇-carbocyclic ring or to represent a -CO-OR₅ group, where R₅ is C₁-C₆-alkyl or C₆-C₁₀-aryl or to represent an -SO₂-OR₆ group, where R₆ is C₁-C₆-alkyl or C₆-C₁₀-aryl or represents a cyano group or a nitro group,
the combinations of substituents
X = F, R = F, R₁ to R₃ = H, Y = -CO-OCH₃ and
X = F, R = CH₃, R₁ to R₃ = H, Y = -CO-OCH₃
being excepted.

2. Fluorinated nitroalkyl compounds according to Claim 1, characterized in that in formula (I) independently of one another X represents fluoro, R represents hydrogen or methyl, R₁ represents hydrogen, methyl or phenyl, R₂ represents hydrogen, methyl, fluoro, chloro, bromo, CN or phenyl, R₃ represents hydrogen, methyl or phenyl and Y represents a -CO-H-, -CO-CH₃-, -COC₂H₅-, -CO-phenyl-, -CO-OCH₃-, -CO-OC₂H₅-, -CO-O-n-butyl, -SO₂-OCH₃-, -SO₂-OC₂H₅- or -SO₂-O-phenyl group.

3. Fluorinated nitroalkyl compounds according to Claim 1, characterized in that in formula (I) Y represents a -CO-R₄ group and R₃ together with the carbonyl carbon of the -CO-R₄ group and the C atoms in between form an unsubstituted saturated C₅ or C₆ ring.

4. Fluorinated nitroalkyl compounds according to Claim 1 or 2, characterized in that in formula (I) X represents fluoro, R represents hydrogen or methyl, R₁ represents hydrogen or methyl, R₂ and R₃ represent hydrogen and Y represents -CO-CH₃, -CN, -CO-OCH₃, -CO-H, -SO₂-OCH₃ or -NO₂.

5. Process for the preparation of fluorinated nitro-alkyl compounds of the formula (Ia) 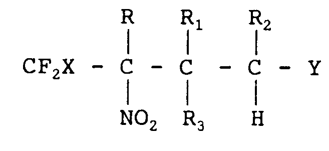 in which
X represents fluoro or hydrogen,
R represents hydrogen, halogen or C₁-C₆-alkyl,
R₁, R₂ and R₃ independently of one another each represent hydrogen, halogen, cyano, substituted or unsubstituted C₁-C₆-alkyl or substituted or unsubstituted C₆-C₁₀-aryl, it also being possible for R₁ and R₂ together with the C atoms to which they are bound to form a substituted or unsubstituted saturated C₅-C₇-carbocyclic ring and
Y represents a -CO-R₄ group where R₄ is hydrogen, C₁-C₆-alkyl or C₆-C₁₀-aryl, it also being possible for R₄ and R₃ together with the C atoms in between to form a substituted or unsubstituted saturated C₅-C₇-carbocyclic ring or to represent a -CO-OR₅ group, where R₅ is C₁-C₆-alkyl or C₆-C₁₀-aryl or to represent an -SO₂-OR₆ group, where R₆ is C₁-C₆-alkyl or C₆-C₁₀-aryl or represents a cyano group or a nitro group,
characterized in that a fluoronitroalkane of the formula (II) 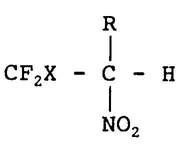 in which
X and R have the meaning given in formula (Ia), is reacted with α,β-unsaturated compounds of the formula (III) 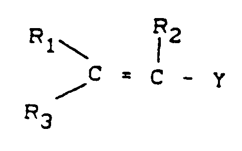 in which
R₁, R₂, R₃ and Y have the meaning given in formula (Ia),
in the presence of alkali metal fluorides and a polar aprotic solvent.

6. Process according to Claim 5, characterized in that, if a compound of the formula (II) where R is hydrogen is used, 3 to 5 moles of the compound of the formula (II) are used per mole of the compound of the formula (III).

7. Process according to Claim 5, characterized in that, if a compound of the formula (II) where R is not hydrogen is used, 1.2 to 3 moles of the compound of the formula (III) are used per mole of the compound of the formula (II).

8. Process according to Claims 5 to 7, characterized in that 0.1 to 100 mole % of alkali metal fluoride is used [based on the compound of the formula (II)].

9. Process according to Claims 5 to 8, characterized in that potassium fluoride and/or caesium fluoride are used.

10. Process according to Claims 5 to 9, characterized in that the reaction is carried out at temperatures in the range from -20°C to the boiling point (at atmospheric pressure) of the lowest boiling component of the reaction mixture.

## Revendications

1. Composés nitroalkyliques fluorés de formule (I) 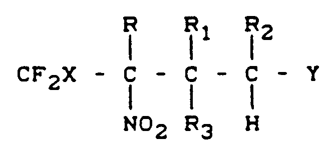 dans laquelle
X représente le fluor ou l'hydrogène,
R est l'hydrogène, un halogène ou un groupe alkyle en C₁ à C₆
R₁, R₂ et R₃ représentent chacun, indépendamment les uns des autres, l'hydrogène, un halogène, un groupe cyano, un groupe alkyle en C₁ à C₆ éventuellement substitué ou un groupe aryle en C₆ à C₁₀ éventuellement substitué, R₁ et R₂ pouvant aussi former conjointement avec les atomes de carbone auxquels ils sont liés, un noyau carbocyclique en C₅ à C₇ saturé éventuellement substitué et
Y est un groupe -CO-R₄ avec R₄ = hydrogène, groupe alkyle en C₁ à C₆ ou groupe aryle en C₆ à C₁₀, R₄ et R₃ pouvant aussi former conjointement avec les atomes de carbone qui se trouvent entre eux, un noyau carbocyclique en C₅ à C₇ saturé éventuellement substitué, ou bien
un groupe -CO-OR₅ avec R₅ = alkyle en C₁ à C₆ ou aryle en C₆ à C₁₀, ou un groupe -SO₂-OR₆ avec R₆ = alkyle en C₁ à C₆ ou aryle en C₆ à C₁₀, ou un groupe cyano ou un groupe nitro,
à l'exclusion des associations de substituants :
X = F, R = F, R₁ à R₃ = H, Y = -CO-OCH₃ et
X = F, R = CH₃, R₁ à R₃ = H, Y = -CO-OCH₃.

2. Composes nitroalkyliques fluorés suivant la revendication 1, caractérisés en ce que dans la formule (I), indépendamment les uns des autres, X représente le fluor, R est l'hydrogène ou le groupe méthyle, R₁ est l'hydrogène, le groupe méthyle ou phényle, R₂ est l'hydrogène, le groupe méthyle, le fluor, le chlore, le brome, le groupe CN ou phényle, R₃ est l'hydrogène, le groupe méthyle ou phényle et Y est un groupe -CO-H, -CO-CH₃, -COC₂H₅, -CO-phényle, -CO-OCH₃, -CO-OC₂H₅, -CO-O-n-butyle, -SO₂-OCH₃, -SO₂-OC₂H₅ ou -SO₂-O-phényle.

3. Composés nitroalkyliques fluorés suivant la revendication 1, caractérisés en ce que, dans la formule (I), Y représente un groupe -CO-R₄ et R₃ forme conjointement avec l'atome de carbone de carbonyle du groupe -CO-R₄ et les atomes intermédiaires de carbone, un noyau pentagonal ou hexagonal saturé non substitué.

4. Composés nitroalkyliques fluorés suivant la revendication 1 ou 2, caractérisés en ce que, dans la formule (I), X est le fluor, R est l'hydrogène ou le groupe méthyle, R₁ est l'hydrogène ou le groupe méthyle, R₂ et R₃ représentent l'hydrogène et Y est un groupe -CO-CH₃, -CN, -CO-OCH₃, -CO-H, -SO₂-OCH₃ ou -NO₂.

5. Procédé de production de composés nitroalkyliques fluorés de formule (Ia) 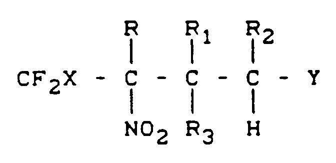 dans laquelle
X représente le fluor ou l'hydrogène,
R est l'hydrogène, un halogène ou un groupe alkyle en C₁ à C₆
R₁, R₂ et R₃ représentent chacun, indépendamment les uns des autres, l'hydrogène, un halogène, un groupe cyano, un groupe alkyle en C₁ à C₆ éventuellement substitué ou un groupe aryle en C₆ à C₁₀ éventuellement substitué, R₁ et R₂ pouvant aussi former conjointement avec les atomes de carbone auxquels ils sont liés, un noyau carbocyclique en C₅ à C₇ saturé éventuellement substitué et
Y est un groupe -CO-R₄ avec R₄ = hydrogène, groupe alkyle en C₁ à C₆ ou groupe aryle en C₆ à C₁₀, R₄ et R₃ pouvant aussi former conjointement avec les atomes de carbone qui se trouvent entre eux, un noyau carbocyclique en C₅ à C₇ saturé éventuellement substitué, ou bien
un groupe -CO-OR₅ avec R₅ = alkyle en C₁ à C₆ ou aryle en C₆ à C₁₀, ou un groupe -SO₂-OR₆ avec R₆ = alkyle en C₁ à C₆ ou aryle en C₆ à C₁₀, ou un groupe cyano ou un groupe nitro,
caractérisé en ce qu'on fait réagir un fluoronitroalcane de formule (II) 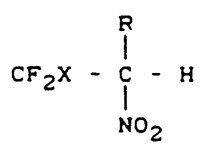 dans laquelle
X et R ont la définition indiquée pour la formule (Ia), avec des composés à non-saturation α,β de formule (III) 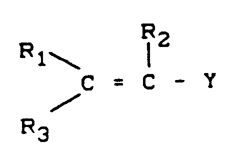 dans laquelle
R₁, R₂, R₃ et Y ont la définition indiquée pour la formule (Ia),
en présence de fluorure de métaux alcalins et d'un solvant aprotique polaire.

6. Procédé suivant la revendication 5, caractérisé en ce que, dans le cas de l'utilisation d'un composé de formule (II), dans laquelle R est l'hydrogène, on utilise 3 à 5 moles du composé de formule (II) par mole du composé de formule (III).

7. Procédé suivant la revendication 5, caractérisé en ce que, dans le cas de l'utilisation d'un composé de formule (II), dans laquelle R n'est pas l'hydrogène, on utilise 1,2 à 3 moles du composé de formule (III) par mole du composé de formule (II).

8. Procédé suivant les revendications 5 à 7, caractérisé en ce qu'on utilise 0,1 à 100 moles % de fluorure de métaux alcalins [par rapport au composé de formule (II)].

9. Procédé suivant les revendications 5 à 8, caractérisé en ce qu'on utilise le fluorure de potassium et/ou le fluorure de césium.

10. Procédé suivant les revendications 5 à 9, caractérisé en ce qu'on opère à des températures comprises dans une plage allant de -20°C au point d'ébullition (à la pression normale) du composant de plus bas point d'ébullition du mélange réactionnel.
